# EUROPEAN PATENT APPLICATION

(11) **EP 1 462 454 A1**
(43) Date of publication of application: **29.09.2004**
(21) Application number: 02788794.2
(22) Date of filing: 11.12.2002
(51) Int. Cl.: C07K 1/00, G01N 33/68, G01N 37/00, B01D 9/02

(54) **ARRAY FOR CRYSTALLIZING PROTEIN, DEVICE FOR CRYSTALLIZING PROTEIN AND METHOD OF SCREENING PROTEIN CRYSTALLIZATION USING THE SAME**

(30) Priority: 11.12.2001 JP 2001376972; 06.08.2002 JP 2002229173
(71) Applicant: Mitsubishi Rayon Co., Ltd., Tokyo 108-8506 (JP)
(72) Inventor: TANAKA, Isao, Sapporo-shi, Hokkaido 006-0812 (JP); WATANABE, Nobuhisa, Sapporo-shi, Hokkaido 065-0028 (JP); TAKEUCHI, Hiroshi, c/o Mitsubishi Rayon Co. Ltd., Yokohama-shi, Kanagawa (JP); AKITA, Takashi, c/o Mitsubishi Rayon Co. Ltd., Yokohama-shi, Kanagawa 23 (JP); NAGATA, Yuichirou, c/o Mitsubishi Rayon Co., Ltd., Tokyo 108-8506 (JP); SUMI, Toshinori, c/o Mitsubishi Rayon Co. Ltd., Yokohama-shi, Kanagawa (JP); NISHIJIMA, Chiharu, Mitsubishi Rayon Co. Ltd., Yokohama-shi, Kanagawa (JP)
(74) Representative: HOFFMANN - EITLE
(86) International application number: PCT/JP2002/012970
(87) International publication number: WO 2003/053998

(57) **Abstract**

The present invention relates to a method of precipitating protein crystals from a protein-containing sample. The present invention also relates to a novel microarray and a novel device for screening for protein crystallization condition. Furthermore, the present invention relates to a method of conveniently and quickly screening for protein crystallization conditions using the microarray or the device having highly integrated and held crystallization conditions even with an extremely small quantity of a sample.

## Description

### TECHNICAL FIELD

The present invention relates to a method of precipitating protein crystals from a protein-containing sample. The present invention relates to a novel microarray and a novel device for screening for protein crystallization condition. Furthermore, the present invention relates to a method of conveniently and quickly screening for protein crystallization conditions from an extremely small quantity of a sample using the microarray or the device having highly integrated crystallization conditions.

### BACKGROUND ART

In recent years, the so-called structural genome science movement has been expanding. This involves the exhaustive analysis of protein structure, based on which mechanisms of living phenomena are investigated. In the field of the structural genome science, further labor saving and increased speed are required for the process of structural analysis. To meet these needs, the development of a method of screening for crystallization conditions quickly and highly efficiently using a minute quantity of a protein sample is expected.

Analysis of a three-dimensional protein structure requires good crystals thereof. Thus, that a large quantity of samples are used and a long time is spent in searching for conditions for preparing such crystals, which is a current problem. That is, existing methods require very complicated procedures and thus have been inefficient, in addition to requiring a large quantity of protein samples.

For this purpose, reagent kits for screening for crystallization have been developed, and circumstances relating to the time for preparing screening reagents required for screening for crystallization conditions have become improved. For example, JP Patent Publication (Kokai) No. 9-89898 proposes an immobilized reagent for screening for protein crystallization conditions, which comprises a solid support having a plural number of types of protein-precipitating agents immobilized thereon. However, including this case, conventional screening for crystallization conditions is conducted using a solution volume of several microliters per condition. The search time for crystallization conditions reaches several hours to few days. Therefore, a further smaller quantity of a sample and a shorter search time for crystallization conditions are expected.

In the meantime, with the above background, various techniques have been developed as methods of crystallizing protein. The major techniques of these methods are the batch method, the vapor diffusion method, and the liquid-liquid diffusion method (or dialysis method).

The "batch method" involves adding the solution of a precipitating agent to a protein solution in driblets so as to make the solution slightly muddy, removing insoluble matters by centrifugation, putting the supernatant in a small test tube or the like, sealing the tube, and then allowing the resultant to stand. Although the operation of this method is convenient, protein crystals are unable to be efficiently obtained thereby. This is because crystals cannot be easily obtained, the quality of the resulting crystals is poor (minute crystals are precipitated), and the active control of crystallization conditions is required.

The "vapor diffusion method" involves placing a droplet of a protein solution containing a precipitating agent in a container containing a buffer (external solution) containing a precipitating agent with a higher concentration, sealing the chamber, and then allowing it to stand. Depending on how the droplet is placed, the hanging drop method and the sitting drop method may be used. The hanging drop method involves spotting a small droplet of a protein solution onto a cover glass and inverting the cover glass over a reservoir so as to seal the reservoir. The sitting drop method involves placing an appropriate droplet stand within a reservoir, spotting a small drop of a protein solution on the droplet stand, and then sealing the reservoir using a cover glass or the like. The vapor diffusion method is thought to be appropriate for screening for wide-ranging crystallization conditions, because the concentration of a protein solution and that of a precipitating agent change over time, and only a small quantity of protein is required for use. A screening method of protein crystallization conditions utilizing the vapor diffusion method has been reported (see the WO 00/60345 pamphlet).

Such a screening method requires an expensive large-scale system involving high throughput in order to examine a large number of conditions using a more minute quantity of a sample.

The "liquid-liquid diffusion method," or "dialysis method," involves bringing a protein solution into contact with a buffer (external solution) containing a precipitating agent via the interface or gel (liquid-liquid diffusion method), or a semipermeable membrane (dialysis method) as a boundary between the solutions, thereby gradually increasing the concentration of the precipitating agent in the protein solution. These methods have the advantages of both the above-described vapor diffusion method and batch method, so that these methods are appropriate for obtaining protein crystals with good quality. However, in these methods, a relatively large volume of protein solution is used, and it is also difficult to make the volume thereof less. Moreover, the procedure therefor is complicated and requires much time, so that it cannot be said to be convenient.

Other than the above-described methods, various techniques have been developed. For example, a technique for crystallizing protein, which utilizes rotation (in other words, gravity) to obtain protein crystals with good quality, has been reported (e.g., JP Patent Publication (Kokai) No. 2002-316899). However, this technique requires a rotation apparatus, so that the whole system becomes excessively large. In addition, since the protein quantity required for this system is approximately 20 µL, the system is inappropriate for a case where only a small quantity of protein is obtained. Furthermore, for example, as an improved vapor diffusion method, a system for crystallizing protein utilizing potential has been reported (e.g., JP Patent Publication (Kokai) No. 2001-213699 and JP Patent Publication (Kokai) No. 2002-179500). With this system, crystals are grown by adsorbing protein molecules on solids utilizing differences in the surface potentials of the solids, and crystals can be grown even when a small quantity of a protein solution is used. However, the system requires the changing of chips (solids) depending on types of protein to be crystallized.

Moreover, a method whereby crystallization is performed in a container provided with a sandwich structure comprising gel containing the protein to be crystallized and gel containing a precipitating agent has been reported (JP Patent Publication (Kokai) No. 6-321700). This technique involves bringing the gel containing the protein into contact with the gel containing the precipitating agent, turning them around so as to diffuse the protein and the precipitating agent between both gels, and causing the protein to react with the agent, thereby crystallizing the protein. However, with this technique, only a reaction condition between 1 type of protein and 1 type of a precipitating agent (concentration and type) can be examined per operation, and thus the method cannot be said to be efficient.

For the reasons described above, a technique to conveniently, highly efficiently, and economically obtain protein crystals in a good state for X-ray diffraction or the like has been desired.

### SUMMARY OF THE INVENTION

An object of the present invention is to provide a method of conveniently and highly efficiently crystallizing protein to obtain good crystals, and a device and a method therefor that enables screening for protein crystallization condition within a short time using a minute quantity of a sample.

As a result of intensive studies to achieve the above objects, we have found that the crystallization conditions of a specific protein can be conveniently and quickly determined using a microarray comprising a plural number of types and concentrations of protein crystallization agents held therein, and using a minute quantity of a sample. Furthermore, we have found that good crystals can be grown by applying a protein-containing sample to gel holding protein crystallization agents, and then bringing the crystallization agents into contact with the protein. Thus, we have completed the present invention.

Hence, the present invention relates to a method of crystallizing protein, which comprises the following steps of:
(a) applying a protein-containing sample to polymer gel holding protein crystallization agents; and
(b) bringing the above protein into contact with the above protein crystallization agents.

In the method of crystallizing protein, the protein-containing sample may contain a protein solubilizing agent and/or an additive, in addition to the protein to be crystallized. Moreover, the polymer gel holding the protein crystallization agents is preferably transparent.

Examples of the protein crystallization agent include a precipitating agent, a pH buffering agent, and any combination thereof. Examples of the precipitating agent include sodium chloride, polyethylene glycol, 2-methyl 2,4-pentanediol, and ammonium sulfate. Examples of the pH buffering agent include sodium acetate trihydrate, potassium phosphate, imidazole, sodium citrate, and sodium cacodylate.

Furthermore, the present invention relates to a microarray for protein crystallization, which comprises at least 10 different types and/or different concentrations of protein crystallization agents held therein per 1 cm² of the microarray surface.

In the microarray for protein crystallization, the protein crystallization agents are preferably held by means of the polymer gel. In addition, the polymer gel holding the protein crystallization agents is preferably transparent.

In addition, the microarray for protein crystallization may comprise a plurality of hollow fibers arrayed therein holding different types and/or different concentrations of the protein crystallization agents in the hollow portions thereof. In this case, the protein crystallization agents can be held by means of the polymer gel that fills the hollow portions.

Examples of the above protein crystallization agent include a precipitating agent, a pH buffering agent, and any combination thereof. Here, examples of the precipitating agent include sodium chloride, polyethylene glycol, and ammonium sulfate. Examples of the pH buffering agent include sodium acetate trihydrate, potassium phosphate, imidazole, sodium citrate, and sodium cacodylate.

Furthermore, the present invention relates to a device for protein crystallization, which is provided with the following (a) and (b):
(a) a microarray for protein crystallization having protein-crystallization-agent-holding portions that comprise at least 10 different types and/or different concentrations of protein crystallization agents held thereon per 1 cm² of the microarray surface; and
(b) a plate having wells that can be filled with a protein-containing sample, and correspond to each protein-crystallization-agent-holding portion of the microarray (a) above for protein crystallization.

In the above (a), the protein crystallization agent is held by means of the polymer gel.

In (a), examples of the protein crystallization agent include a precipitating agent, a pH buffering agent, and any combination of these. In addition in (a), the microarray for protein crystallization may comprise a plurality of hollow fibers arrayed therein holding different types and/or different concentrations of the protein crystallization agents in the hollow portions thereof. In this case, the protein crystallization agents can be held by means of the polymer gel filling the hollow portions.

Furthermore in (b), the capacity of each well is preferably 1 µL or less. Furthermore in (b), when the plate is superposed on the microarray (a) for protein crystallization, the superposed portion corresponding to each protein-crystallization-agent-holding portion of the array is preferably transparent. Moreover, the plate is preferably made of an optically transparent material, such as a transparent resin or glass.

In (b) above, the plate can have a structure wherein a plate material perforated regularly to have a plurality of holes is adhered to a substrate. Here, the substrate is preferably prepared from an optically transparent material, such as a slide glass. Moreover, the plate material preferably is made of material selected from the group consisting of metal, resin and rubber.

The microarray (a) above for protein crystallization may be fixed on a support. Here, the support is preferably made of an optically transparent material such as a slide glass. In addition, marking can be made on the support, so as to determine a position to which the microarray (a) for protein crystallization is fixed.

Furthermore, in the device for protein crystallization, a spacer can be placed between the support and the plate (b), where the spacer has space into which the microarray (a) for protein crystallization fixed on the support can be accommodated. Here, the spacer is made of material selected from the group consisting of metal, resin, and rubber.

Moreover, in the above microarray for protein crystallization or the above device for protein crystallization, the present invention relates to a method of screening for protein crystallization condition, which comprises a step of precipitating protein by bringing the protein crystallization agents into contact with a protein-containing sample on and/or in the microarray. Here, the protein-containing sample may further contain a protein solubilizing agent and a protein stabilizing agent such as a reducing agent, in addition to protein to be screened for.

In addition, the above screening method can be conducted under a plurality of temperature conditions.

Furthermore, the volume of the protein-containing sample solution used in the above screening method is preferably 1 µL or less per type or concentration of the protein crystallization agent.

Furthermore, in the above device for protein crystallization, the present invention relates to a method of screening for protein crystallization conditions, which comprises a step of precipitating protein by superposing the wells of (b) filled with the protein-containing sample onto the protein-crystallization-agent-holding portions holding the protein crystallization agents on the microarray (a), so as to bring the protein crystallization agents into contact with the protein.

The terms relating to the present invention are as defined below.

The term "protein" used in this specification includes natural or synthetic peptides, polypeptides, protein, and protein complexes. These substances can be purified by extraction and isolation from natural or synthetic materials, or generation by genetic engineering techniques, chemical synthesis techniques, or the like, and then a combined use of general purification methods such as solvent extraction, column chromatography, liquid chromatography, and the like.

The term "crystallization" used in this specification refers to obtaining crystals by growing or precipitating crystals from a protein solution.

The term "protein-containing sample" used in this specification refers to a sample containing protein to be crystallized or protein whose crystallization conditions are to be specified.

The term "protein crystallization agent" used in this specification means a compound that acts to lower the solubility of protein. Examples of the protein crystallization agent include a precipitating agent, a pH buffering agent, other additives.

The term "hold" used in this specification means to immobilize the protein crystallization agent to polymer gel or a microarray.

The term "polymer" used in this specification includes any natural polymers (e.g., biopolymers) and synthetic polymers.

The term "polymer gel" used in this specification means polymer gel prepared by polymerizing or gelatinizing a polymerizable monomer or a solution thereof by a polymerization reaction, or polymer gel prepared by causing a solution to lose its flow property by adding a synthetic polymer or a natural polymer.

The term "apply" used in this specification refers to, for example, adding a protein-containing sample dropwise to protein-crystallization-agent-holding portions, filling the portions with the sample manually or mechanically using a syringe or the like, or immersing a microarray in a protein-containing sample.

The term "bring...into contact with" used in this specification refers to, for example, bringing a protein-containing sample near to a protein crystallization agent to a degree such that a reaction can occur between the two.

The term "microarray" used in this specification refers to, generally, a microarray having a configuration wherein many substances for reaction are arranged in order to conduct many reactions on a minute support.

The term "hollow portion" used in this specification means, for example, a hollow portion within a tubular or a hollow fiber.

The term "hollow fiber" used in this specification means a tubular or a straw-shaped fiber whose inside is hollow.

The term "protein-crystallization-agent-holding portion" used in this specification refers to a portion holding a protein crystallization agent. Examples of such a portion in the present invention include polymer gel or array portions of a microarray.

The term "fill" used in this specification means to fill a portion by putting a protein-containing sample into the portion.

The term "well" used in this specification means a part that is dented compared with the surroundings. Examples of such a well include those having various shapes such as holes and grooves.

The term "optically transparent material" used in this specification means a material that is transparent and through which light is transmittable.

The term "(to create a) hole" used in this specification means to create holes and the resulting holes.

The term "regularly" used in this specification refers to array in an orderly manner holes within a frame with a certain size so that the number of fibers or the number of holes become fixed.

The term "plate material" used in this specification means a member for preparing a plate, which is made of material and has a shape that enable the formation of wells that can be filled with a protein-containing sample.

The term "substrate" used in this specification means a member to which a plate material is adhered in order to prepare a plate.

The term "adhere" used in this specification means to bring and keep members into contact with each other.

The term "support" used in this specification means a member to which a microarray for protein crystallization is fixed.

The term "fix" used in this specification means to secure an object to a position.

The term "marking" used in this specification means to make a mark that is an indication (label).

The term "can be accommodated" used in this specification means that a member can be placed into another member or between members.

The term "space" used in this specification means an opening or a hole of a member.

The term "spacer" used in this specification means a member to be used to provide a distance between members.

The term "screening" used in this specification means the step of selecting conditions for protein to crystallize, and the step of narrowing appropriate conditions from among many conditions.

The present invention is explained in detail as follows. This application claims a priority from JP Patent Application No. 2001-376972 filed on December 11, 2001, and JP patent application No. 2002-229173 filed on August 6, 2002, and includes part or all of the contents as disclosed in the specifications and drawings of the above patent applications.

The present invention relates to a method of crystallizing protein, which comprises applying a protein-containing sample to polymer gel holding protein crystallization agents, and bringing the protein into contact with the protein crystallization agents utilizing the diffusion phenomenon of the gel. Various interactions intricately affect protein crystallization. To enable gradual and slow changes in these various interactions, the present invention slowly brings the protein into contact with the protein crystallization agents utilizing the diffusion phenomenon of the gel, thereby creating a state where the interaction between them and other interactions change gradually. As a result, by the method of crystallizing protein, protein crystals with good quality can be precipitated conveniently and highly efficiently.

Furthermore, the present invention relates to a method of screening for protein crystallization condition utilizing a microarray for protein crystallization or a device for protein crystallization, which comprises protein crystallization agents held therein. Protein crystallization is affected by parameters such as the purity and the concentration of the protein, the type and the concentration of a precipitating agent, the type and the concentration of a buffering agent or salt, pH, and temperature. The crystallization conditions for a specific protein are determined by a combination of these parameters. The present invention conveniently and quickly determines the optimum conditions from many assumed crystallization conditions utilizing the microarray wherein these parameters are highly integrated and using a small quantity of a protein sample.

### 1. Method of crystallizing protein

The method of crystallizing protein comprises applying a protein-containing sample to polymer gel holding protein crystallization agents, and bringing the protein into contact with the protein crystallization agents in the gel. Specifically, the method of crystallizing protein comprises the steps of (a) applying a protein-containing sample to polymer gel holding protein crystallization agents, and (b) bringing the protein into contact with the protein crystallization agents.

### Step (a): Applying to polymer gel

In step (a) of the method of crystallizing protein, a protein-containing sample is applied to polymer gel holding protein crystallization agents. Here, the protein-containing sample may further contain a protein solubilizing agent that help protein be dissolved, a stabilizing agent such as a reducing agent, or the like, in addition to protein to be crystallized. As the protein solubilizing agent, for example, a surfactant or the like that dissolves membrane protein can be exemplified. Furthermore, the polymer gel holding the protein crystallization agents can be prepared as described below.

Types of polymer gel that can be used in the present invention are not specifically limited. For example, gel that can be used herein is prepared by conducting, in an aqueous medium, for example, co-polymerization using 1, 2, or more types of monomers such as acrylamide, N, N-dimethylacrylamide, N-isopropylacrylamide, N-acryloylaminoethoxyethanol, N-acryloylaminopropanol, N-methylolacrylamide, N-vinylpyrrolidone, hydroxyethylmethacrylate, (meta)acrylic acid, or allyl dextrin, and multifunctional monomers, such as methylenebis(meta)acrylamide, polyethylene glycol di(meta)acrylate, or the like. Additional examples of polymer gel that can be used in the present invention include gel such as agarose, alginic acid, dextran, polyvinyl alcohol, and polyethylene glycol, or gel prepared by cross-linking these.

To fill any container (e.g., microarray) with gel, a liquid containing monomers such as acrylamide, multifunctional monomers, and an initiator, which are gel components, is poured into the chamber for polymerization and gelatinization to proceed. Gelatinization may also be conducted by conducting co-polymerization in the absence of the multifunctional monomers and then using a cross-linking agent, in addition to a method that involves conducting co-polymerization in the presence of the multifunctional monomers. Moreover, in the case of agarose gel, gelatinization may be conducted by lowering temperature.

A method for causing the polymer gel to hold the protein crystallization agents is not specifically limited. Here, "hold" means to immobilize the protein crystallization agents in the polymer gel. For example, the protein crystallization agents and the above polymerizable monomers may be previously mixed and introduced into an appropriate chamber, so as to cause the formation of the polymer gel through the polymerization process. Thus, the protein crystallization agents can be immobilized. Although the protein crystallization agents to be held by the polymer gel differ depending on the type and the concentration of protein to be crystallized, persons skilled in the art can select appropriate protein crystallization agents, appropriately set the concentrations and the quantities thereof, and thus can cause the polymer gel to hold the agents.

Alternatively, porous particles or the like may be impregnated with the protein crystallization agents, and then the particles can be included in the polymer gel.

For the protein crystallization agent, for example, conditions similar to those for commercially available Grid Screen™, Crystal Screen™ I & II, and Wizard™ I & II can be used.

To make it possible to observe over time how crystallization proceeds under a microscope or the like, the polymer gel holding the protein crystallization agents is preferably transparent. "Transparent" does not always mean optical transmittancy of 100%. The polymer gel may be transparent to a degree such that how crystallization proceeds is observable.

For example, when NaCl is held in gel, the gel is preferably prepared from acrylamide, 2-acrylamide-2-methylpropanesulfonic acid, or methacryloyl oxyethyltrimethylammonium chloride. Furthermore, by appropriate selection of the above monomers and the like depending on the types of the protein crystallization agents involved, the polymer gel holding the transparent protein crystallization agents can be obtained.

To the above-prepared polymer gel holding the protein crystallization agents, a protein-containing sample is applied. The protein-containing sample can be applied by any technique. For example, the protein-containing sample may be applied by adding it dropwise to the polymer gel holding the protein crystallization agents, filling the polymer gel with the sample manually or mechanically using a syringe or the like, or immersing the polymer gel in the protein-containing sample. The quantity of the protein-containing sample to be applied is set considering protein crystallization conditions and the like.

### Step (b): Contact of protein with protein crystallization agent

As described above, after the protein-containing sample is applied to the polymer gel holding the protein crystallization agents, the protein comes into contact and reacts with the protein crystallization agents. In the method of crystallizing protein, the protein crystallization agents gradually shift from within the gel to portions corresponding to the protein-containing sample, or the protein-containing sample gradually shifts into the gel holding the protein crystallization agents. Thus, the crystallization reaction occurs little by little. Accordingly, interaction between the protein and its surrounding environment (e.g., involving the presence of the protein crystallization agents and pH levels), which is the crystallization mechanism, gradually changes, so that crystals with good quality are precipitated.

After the application of the protein-containing sample, the polymer gel is allowed to stand in a state of being sealed or in the open air under appropriate temperature conditions for a time period sufficient for the protein to be precipitated.

The time period sufficient for the protein to be precipitated differs depending on a specific protein, concentration, crystallization conditions, and the like, and ranges from approximately 1 hour to 10 days. The appropriate temperature conditions also differ depending on a specific protein, concentration, crystallization conditions, and the like, and ranges from approximately 4°C to 30°C.

After the time period sufficient for the protein to be precipitated has passed, the state of precipitation of protein crystals is observed under an optical microscope, X-ray diffractometer, or the like. In the method of crystallizing protein, a combination of known systems for monitoring protein crystallization in the polymer gel can be used. For example, the way that crystals are precipitated can be recorded by taking pictures using a CCD camera mounted in a microscope, and then the images are processed, so that whether crystallization is successful or not can be determined at a high speed.

As described above, the method of crystallizing protein comprises causing a protein-containing sample with diffusion in the gel to gradually come into contact with and react with protein crystallization agents, thereby crystallizing the protein. Therefore, according to the present invention, with only a simple operation of applying a protein-containing sample, protein crystals with good quality, which is appropriate for use in X-ray analysis or the like, can be obtained. Moreover, according to the method of crystallizing protein, as described later, for example, polymer gel holding the protein crystallization agents is prepared on a microarray, and then the protein can be crystallized on the array. In this case, the quantities of the protein to be used and the protein crystallization agent can be reduced. Even with the use of a small quantity of protein, good crystals can be obtained with high efficiency. Furthermore, the operation is convenient, so that the method is also appropriate for screening for protein crystallization conditions.

### 2. Microarray for protein crystallization

The microarray for protein crystallization comprises at least 10 different types and/or different concentrations of protein crystallization agents held therein per 1 cm² of the microarray surface.

The microarray used in the present invention is not limited to a microarray being made of specific material, a shape, or the like, as long as it has a general configuration wherein many substances for reaction are arranged in order. Examples of materials used for the microarray include glasses, resins, and polymer gel. In addition, a complex prepared by combining these materials can also be used. For the microarray of the present invention, materials with high optical transparency are preferably used in order to easily confirm protein precipitation.

In the microarray of the present invention, the shape of the array is not specifically limited, as long as it meets requirements such that upon the application of a protein-containing sample, the sample is easily and uniformly dispensed to portions holding the protein crystallization agents (hereinafter referred to as "protein-crystallization-agent-holding portions"), and that this apportion can be performed quickly and efficiently without obstructing observation of crystal precipitation. Examples of the shape of the array include a smooth shape having no extreme roughness, a shape having grooves formed thereon for apportioning a protein-containing sample to the protein-crystallization-agent-holding portions, a shape having, for example, circular or quadrangular reservoirs as the protein-crystallization-agent-holding portions, and a shape using these in combination.

To prevent a plurality of different types and different concentrations of protein crystallization agents held in the microarray from mixing with each other, a supplemental member for partition, which is made of material having no permeability for a solvent, is used integrated with the microarray, so as to be able to create the protein-crystallization-agent-holding portions. As such a supplemental member, for example, members prepared by molding plate-shaped resins into a matrix shape, or non-porous hollow fibers prepared using polyethylene or the like can be used.

The number of the protein-crystallization-agent-holding portions is preferably 10 or more, and more preferably 100 or more per 1 cm² of the microarray.

Furthermore, the microarray used in the present invention can have any shape that does not obstruct observation of crystal precipitation, such as circular, square, and rectangular shapes. The thickness of the microarray can be appropriately selected considering improvement in the efficiency of crystallization and ease and speed of observation of crystal precipitation. For example, the microarray can have a thickness between 0.1 and 5 mm, and preferably between 0.5 and 3 mm.

Next, as an example of the microarray used in the present invention, a microarray comprising a plurality of tubulars arrayed therein is explained in detail.

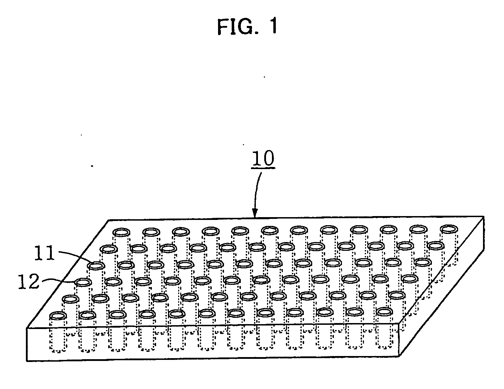

An example of the microarray comprising a plurality of tubulars arrayed therein has a shape of a hollow-fiber-arrayed-thin-section 10 provided with a plurality of hollow fibers 12 each having a hollow portion 11, as shown in Fig. 1. The hollow fibers used in the present invention are preferably non-porous, and examples of the hollow fibers include those formed of monopolymers of methacrylate-based monomers such as methyl methacrylate, ethyl methacrylate, and butylmethacrylate; acrylate-based monomers such as methylacrylate and ethylacrylate; or copolymers thereof; polystyrene; polyethylene; norbornene/ethylene copolymers; polyethylene terephthalate; polycarbonate; or glass.

To obtain the thin sections of the hollow-fiber-array as shown in Fig. 1, fibers are arrayed in parallel at certain intervals. In addition, to increase the number of the protein-crystallization-agent-holding portions per unit area, the external diameter of the hollow fiber is preferably 1 mm or less, and more preferably 0.5 mm or less. Furthermore, in the microarray of the present invention, in view of causing the hollow portions to hold the protein crystallization agents, the internal diameter of the hollow fibers is preferably 10 µm or more, and more preferably 50 µm or more.

The hollow fibers may be used with their inner wall surfaces untreated, or may be used with their inner wall surfaces treated with plasma or radiation such as γ-rays and electron beams, if necessary. Furthermore, a reactive functional group may be introduced into the hollow fibers, if necessary.

The hollow fibers prepared as described above can form a basic unit to compose the microarray of the present invention comprising a plurality of the hollow fibers arrayed therein. Then, these hollow fibers are bundled, and then adhered, thereby forming a fiber-array (three-dimensional-array).

The above three-dimensional-array is cut in a direction so as to cross with the fiber axis, and is preferably cut in a direction vertically to the fiber axis using a device for preparing sections, such as a microtome, so that a thin section having cross sections of the hollow-fiber-arrayes can be obtained (Fig. 1). As for the thickness of the thin section, the thickness is generally between 100 and 5000 µm, and preferably between 500 and 3000 µm.

At this time, the hollow fibers are arrayed regularly, and then made to adhere using a resin adhesive or the like, so that, for example, a hollow-fiber-array wherein the hollow fibers are regularly arrayed in order vertically and horizontally can be obtained. The shape of the hollow-fiber-array is not specifically limited, and is generally formed into, for example, a square, rectangular, circular, or helical shape by regularly arraying the fibers.

"Regularly (array)" refers to an array chip in order so that the same number of the fibers is always contained in a frame with a certain size. For example, when fibers having a diameter of 1 mm are bundled and arrayed so as to form a square cross section 10 mm in length and 10 mm in width, 10 fibers are contained within one side of the square frame (1 cm²). After these 10 fibers are bundled and placed in a row so as to form one layer of sheet, these sheets are stacked to form 10 layers of sheet. As a result, 10 fibers can be arrayed lengthwise and 10 fibers can be arrayed breadthwise, that is, 100 fibers can be arrayed in total. However, the technique for arraying fibers regularly is not limited to the above wherein sheets are multilayered.

The number of fibers to be bundled in the present invention is 10 fibers or more, and preferably 100 fibers or more, and the number thereof can be appropriately set depending on purposes.

Regarding details about the above-described microarray comprising a plurality of hollow fibers arrayed therein, see the WO 00/53736 pamphlet.

The thin section of the hollow-fiber-arrays prepared as described above has a plurality of the protein-crystallization-agent-holding portions, and the protein crystallization agents held therein are not mixed, so that the this section is preferred as the microarray of the present invention for protein crystallization.

The microarray of the present invention for protein crystallization holds the protein crystallization agents directly or by means of gel or the like. The "protein crystallization agent" means a compound that acts to lower the solubility of protein as described above, and examples of the agent include a precipitating agent, a pH buffering agent, and other additives.

As the precipitating agent, salt (e.g., sodium chloride, ammonium sulfate, magnesium chloride, or calcium chloride), polyethylene glycol with a molecular weight between 400 and 20000, and organic solvents (e.g., 2-methyl-2,4-pentanediol, ethanol, methanol, isopropanol, and dioxane) can be used alone, or a plurality of them can be used in combination.

As the pH buffering agent, sodium acetate trihydrate, potassium phosphate, imidazole, sodium citrate, sodium cacodylate, and the like can be used alone, or a plurality of these components can be mixed and used.

As the additives, monovalent salt, divalent salt, glycerol, surfactant, and the like can be used.

Moreover, as the protein crystallization agent, those based on known documents and those prepared based on propositions made by users may be used.

In the present invention, when a plurality of protein crystallization agents is used, the number thereof is appropriately determined depending on different parameters, that is, a combination of concentrations and types of protein crystallization agents.

The present invention enables easy and quick screening for appropriate protein crystallization conditions from a large number of pre-set protein crystallization conditions by applying a large number of protein crystallization agents set according to such different parameters to the same experimental system (microarray).

In the microarray of the present invention, 10 or more protein-crystallization-agent-holding portions (e.g., hollow portions of hollow fibers) are arrayed. Thus, 10 or more concentrations and types, and approximately a maximum of 1000 concentrations and types, can be combined. Accordingly, protein crystallization conditions can be screened for exhaustively.

The concentrations of protein crystallization agents to be held in the microarray of the present invention depend on types of the protein crystallization agents, for example, in the case of polyethylene glycol as a precipitating agent, 5 to 50% by volume, and preferably 10 to 35% by volume; and in the case of a pH buffering agent, 0.05 to 0.5 mol/L, and preferably 0.1 to 0.2 mol/L. An object of the present invention is to cause protein crystallization agents at a plurality of concentrations to be held on a microarray, and the protein crystallization agents at multi-levels of concentrations within the above range are preferably used. Specifically, for example, when sodium chloride is used as a precipitating agent, on the same microarray, for example, 5-grade, 10-grade, or 20-grade dilution series, or the like within a range between 0.5 and 4.0 mol/L are prepared. Regarding the concentrations of protein crystallization agents to be held, the concentration ranges and the concentration grades, or combinations thereof, can be appropriately determined by persons skilled in the art considering types of the protein crystallization agents and the like.

The quantities of protein crystallization agents to be held on the microarray are not specifically limited, and can be appropriately determined.

The above-described protein crystallization agents with a plurality of types and concentrations are held on the microarray. The term "hold" means to immobilize the protein crystallization agents on the microarray.

A method for causing protein crystallization agents to be held in the protein-crystallization-agent-holding portions in the microarray, that is, reservoirs such as grooves or the hollow portions of the hollow fibers, is not specifically limited. For example, protein crystallization agents may be directly held in the protein-crystallization-agent-holding portions in the microarray, or held by means of polymer gel or the like. Moreover, the protein crystallization agents impregnated in porous particles or the like can be included in polymer gel. Furthermore, a method that can also be employed herein involves mixing the protein crystallization agents with polymerizable monomers and previously introducing the resultant into the protein-crystallization-agent-holding portions of the microarray, and then forming polymer gel through a polymerization process, so as to conduct immobilization.

The types of gel that can be used in the present invention are not specifically limited. For example, gel that can be used herein can be prepared by copolymerization using 1, 2, or more types of monomers such as acrylamide, N,N-dimethylacrylamide, N-isopropylacrylamide, N-acryloylaminoethoxyethanol, N-acryloylaminopropanol, N-methylolacrylamide, N-vinylpyrrolidone, hydroxyethylmethacrylate, (meta)acrylic acid, or allyldextrin, and multifunctional monomers, such as methylenebis(meta)acrylamide, polyethyleneglycoldi (meta)acrylate, or the like, for example, in an aqueous medium. Further examples of gel that can be used in the present invention include gel such as agarose, alginic acid, dextran, polyvinyl alcohol, and polyethylene glycol, or gel prepared by crosslinking the same.

To fill the protein-crystallization-agent-holding portions of the microarray with gel, a liquid containing monomers such as acrylamide, multifunctional monomers, and an initiator, which are gel components, are injected into the protein-crystallization-agent-holding portions of the microarray, followed by polymerization and gelatinization. In addition to a method that involves conducting co-polymerization in the presence of multifunctional monomers, gelatinization may also be conducted using a cross-linker after co-polymerization in the absence of multifunctional monomers. Furthermore, in the case of agarose gel, gelatinization may be conducted by lowering temperature.

As described above, the microarray of the present invention for protein crystallization can be obtained. The prepared microarray is preferably stored in a container that is sealed well so as to prevent contact with the open air. Examples of material for a container for storage include polymer materials having a small degree of gas or water permeability, or glass and metal. The microarray is preferably stored at a low temperature. When the microarray is stored for particularly a long time period, it can also be freezed.

### 3. Device for protein crystallization

The device for protein crystallization is provided with (a) a microarray for protein crystallization having protein-crystallization-agent-holding-portions, which comprises at least 10 different types and/or different concentrations of protein crystallization agents held therein per 1 cm² of the microarray surface; and (b) a plate, which has wells that can be filled with a protein-containing sample, corresponding to each protein-crystallization-agent-holding-portion of the microarray (a) above for protein crystallization.

Configuration of such a device enables the achievement of further smaller quantities of required protein samples, simplification of experimental protocols for crystallization, and smaller space required for experiments by an ultrahigh degree of integration. Moreover, it can ease quantification of a protein-containing sample and observation of a state of crystallization under a microscope.

### (a) Microarray for protein crystallization

In the device for protein crystallization, as a member (a), the microarray for protein crystallization described in the above section "2. Microarray for protein crystallization" can be used.

Furthermore, the microarray (a) for protein crystallization may be fixed onto a support such as a flat plate. The support that can be used herein is not specifically limited, as long as it has a shape and is made of material to which the microarray can be fixed. For example, as the material for the support, glass, resin, and metal can be exemplified. The material for the support can be appropriately selected from them, and one type alone or 2 types or more of material can be used in combination. The support to which the microarray for protein crystallization is fixed is preferably prepared from, in particular, an optically transparent material (e.g., slide glass). By the use of the optically transparent material, the thus generated crystals can be quickly and conveniently confirmed under a microscope while keeping the crystals in the device, and the growth process of the crystals can be observed over time under a microscope.

Furthermore, it is preferred to make a marking on the above support so as to determine a position to which the microarray for protein crystallization is fixed. A method for marking is not specifically limited, as long as the microarray for protein crystallization can be fixed precisely. An appropriate method involves forming a frame pattern having an external shape along the circumference of the microarray while adhering it to the support. In this case, the material for the frame pattern for marking is not specifically limited, and stainless steel is appropriate.

### (b) Plate

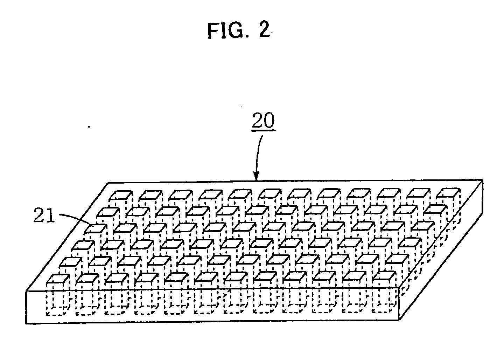

In the device for protein crystallization, the member (b) is a plate having wells that can be filled with a protein-containing sample, corresponding to each protein-crystallization-agent-holding portion of the microarray (a) above for protein crystallization. This plate is typically shown in Fig. 2. In Fig. 2, wells 21 that can be filled with a protein-containing sample are formed on a plate 20. However, the plate configuration shown in this figure is an example, and the configuration is not limited thereto.

The plate that can be used in the present invention is not specifically limited, as long as it has a shape and is made of material so that it can be superposed on the microarray (a) for protein crystallization, and can be selected or designed appropriately in consideration of the shape of the microarray (e.g., with a smooth shape without any roughness). As the material for the plate, glass, resin, metal, and the like can be exemplified. The material for the plate can be appropriately selected from them, and one type alone or 2 types or more of material can be used in combination. To observe a state of protein crystallization, the plate has preferably transparent (optically transparent) portions that are superposed on the microarray (a) for protein crystallization, corresponding to each protein-crystallization-agent-holding portion of the array chip. Hence, the plate is particularly preferably made of an optically transparent material (e.g., transparent resin or glass).

On the plate (b), the wells that can be filled with a protein-containing sample are formed. The plate has at least 10 wells per 1 cm² of the plate, corresponding to the protein-crystallization agent-holding portions of the microarray (a) for protein crystallization. The method for forming the wells is not specifically limited. For example, at least 10 holes may be regularly created per 1 cm² of any plate material, and then the plate material may be adhered to a substrate, thereby forming the wells. The plate material is not limited, and can be made of material such as metal, resin, and rubber. In particular, the plate material is preferably made of stainless steel. As a technique to create holes on the plate material, a technique such as injection molding, drilling, laser processing, optical molding, or etching can be employed. The substrate to which the plate material is adhered is preferably transparent (optically transparent), because the substrate portion is superposed in a manner corresponding to each protein-crystallization-agent-holding portion of the array chip. Hence, the substrate is preferably made of an optically transparent material (e.g., slide glass).

The shape of the well to be formed is not specifically limited, as long as the wells can be arranged so as to be superposed in a manner corresponding to each protein-crystallization-agent-holding portion of the microarray, and can be filled with a protein-containing sample. For example, in terms of ease of formation of the well, the wells are preferably lattice-shaped. The capacity of the well is not specifically limited, as long as it is sufficient for filling the well with a protein-containing sample. When reduction of protein quantity, which is one of the purposes of the present invention, is taken into consideration, the capacity of the well is preferably 1 µL or less, more preferably 100 nL or less, and further preferably 50 nL or less.

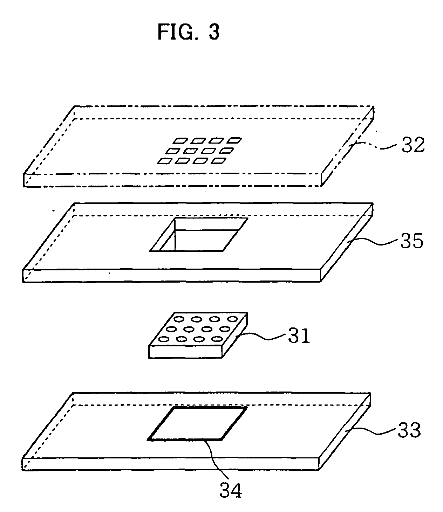

In the device for protein crystallization, the above device (a) for protein crystallization is used with the plate (b) superposed thereon. Fig. 3 typically shows how the microarray for protein crystallization and the plate are arranged on the device for protein crystallization. In Fig. 3, a microarray 31 for protein crystallization is fixed on a support 33 on which a marking 34 is made to determine the position for this fixation. In the device for protein crystallization, a spacer 35 is preferably placed between the support 33 and the plate 32, which has a space into which the microarray 31 for protein crystallization fixed on the support 33 can be accommodated. The spacer 35 is a supplemental member for fixing the microarray 31 fixed on the support 33 at a fixed position, and by which it becomes possible to place each well part of the plate 32 in a manner corresponding to each hollow portion of the microarray 31.

The shape and the material for the spacer are not specifically limited, as long as the spacer is appropriate for use in combination with other components of the device for protein crystallization (e.g.; the microarray for protein crystallization, the plate, or the support). Examples of material that can be appropriately used include metal, resin, and rubber. The spacer 35 is preferably made of stainless steel in terms of ease for the formation of the space, strength, salt tolerance, and the like.

The size of the space is not particularly limited as long as it is sufficient for the microarray 31 for protein crystallization to be accommodated. A particularly preferred size enables the microarray 31 for protein crystallization to be accommodated without any gap. Furthermore, the thickness of the spacer preferably enables the microarray 31 for protein crystallization fixed on the support 33 to be in contact with the plate 32 without any gap.

The device for protein crystallization comprises a combination of the microarray for protein crystallization having various protein crystallization conditions integrated on a sheet of the array chip, and the plate having wells that can be filled with a protein-containing sample. Hence, the plate is filled with a protein-containing sample, and then the plate is superposed on the microarray for protein crystallization, so that the protein-crystallization-agent-holding portions on the array chip and the wells filled with the protein-containing sample are superposed corresponding to each other, thereby bringing the protein into contact with and to react with the protein crystallization agents. Here, a technique for filling the wells with a protein-containing sample can be a known technique, such as a technique that involves simply pouring the protein-containing sample into the wells. In addition, the order of superposing (up or down) the microarray for protein crystallization and the plate is not specifically limited.

In the device for protein crystallization, partitions for each protein-crystallization-agent-holding portion are specified by the use of the plate, so as to be able to prevent samples or other substances from shifting or mixing (contamination) among arrays, when a protein-containing sample is applied to the arrays.

### 4. Screening for crystallization conditions

The method of the present invention for screening for protein crystallization conditions comprises, using the above microarray for protein crystallization or the above device for protein crystallization, a step of precipitating protein by bringing protein crystallization agents into contact with a protein-containing sample on and/or in the microarray.

In the present invention, a protein-containing sample is a sample containing protein whose crystallization conditions are to be specified. Examples of the protein include natural or synthetic peptides, polypeptides, protein, and protein complexes. These substances are preferably extracted or isolated, or generated by a genetic engineering technique, a chemical synthesis technique, or the like from natural or synthetic sources, and then previously purifying protein to be screened for by a combined use of general purification methods such as solvent extraction, column chromatography, and liquid chromatography.

Since protein concentration and purity in a protein-containing sample are also factors in protein crystallization conditions, protein-containing samples may be prepared at several grades of concentration and purity, and then allowed to react with protein crystallization agents. For example, protein concentration is preferably changed at several grades within a range between 5 and 30 mg/ml. In addition, the quantity of a protein-containing sample for the reaction with protein crystallization agents can be appropriately changed depending on array size.

The protein-containing sample may contain, in addition to protein to be screened for, a protein solubilizing agent to help protein be dissolved, a protein stabilizing agent such as a reducing agent, or the like. As the protein solubilizing agent, for example, a surfactant to dissolve, for example, membrane protein can be exemplified.

The method of the present invention of screening for protein crystallization conditions comprises the step of precipitating protein by bringing the above protein-containing sample into contact with the protein crystallization agents in the above microarray or the above device. Here, "bringing...into contact" refers, for example, to add a protein-containing sample dropwise to the protein-crystallization-agent-holding portions, filling the portions with the sample manually or mechanically using a syringe or the like, or immersing the microarray in the protein-containing sample.

After the protein crystallization agents are allowed to react with the protein-containing sample in the microarray or the device, the microarray or the device is allowed to stand in a state of being sealed or in the open air under a specific temperature condition for a time period sufficient for protein to be precipitated.

The time period that is sufficient for protein to be precipitated differs depending on a specific protein, concentration, crystallization conditions, and the like, and ranges from approximately 1 hour to 10 days. When no crystals are precipitated even after 30 days or more have passed, the crystallization conditions therefor are determined to be inappropriate.

Since the temperature condition is a factor in protein crystallization conditions, the screening method may be conducted by changing the temperature condition. The temperature condition is preferably set at several grades such as at 4°C, 15°C, 18°C, or 22°C.

After time period sufficient for protein to be precipitated has passed, the way that protein crystals are precipitated is observed using, for example, an optical microscope or an X-ray diffractometer.

When protein crystallization conditions are screened for using the microarray or the device of the present invention, known systems for monitoring protein crystallization in the microarray or the device can be used in combination. Specifically, the way that crystals are precipitated in many crystallization conditions in the microarray or the device can be recorded by, for example, taking pictures using a CCD camera mounted in a microscope, and then processing the images, so that whether crystallization is successful or not can be determined at a high speed.

When protein crystals are confirmed, the confirmed conditions are determined as crystallization conditions for the protein. Alternatively, to narrow down conditions to more detailed protein crystallization conditions, a second-stage screening method can be conducted wherein protein crystallization conditions are set within a narrower range.

When protein crystallization conditions are screened for in this manner, using the crystallization conditions, protein is precipitated from the original protein sample by the above-described method for crystallizing protein or a method for crystallizing protein that is generally employed in the art such as a hanging drop method, a sitting drop method, a dialysis method, or a batch method.

The microarray or the device for protein crystallization of the present invention comprises a plurality of protein crystallization conditions integrated therein. The use of the microarray or the device of the present invention enables screening for appropriate conditions simultaneously from a plurality of protein crystallization conditions using a small quantity of protein, and thus is useful in easily and quickly determining protein crystallization conditions.

### BRIEF DESCRIPTION OF THE DRAWINGS

Fig. 1 is an oblique view typically showing a hollow-fiber-arrayed thin section containing gel, which is an example of a microarray for protein crystallization according to the present invention.
Fig. 2 is an oblique view typically showing a plate that is superposed on a microarray for protein crystallization in a device for protein crystallization.
Fig. 3 is an oblique view typically showing arrangement of the microarray for protein crystallization and the plate in the device for protein crystallization.
Fig. 4 is a microphotograph showing a lysozyme in a crystal state using sodium chloride as a crystallization agent on the hollow fiber-arrayed thin section containing gel.

### Explanation of symbols

- 10: Hollow-fiber-arrayed thin section
- 11: Hollow portion
- 12: Hollow fiber
- 20: Plate
- 21: Well
- 31: Microarray for protein crystallization
- 32: Plate
- 33: Support
- 34: Marking
- 35: Spacer

### BEST MODE OF CARRYING OUT THE INVENTION

The present invention will be described in more detail with reference to the following examples, but the present invention is not limited to these examples.

### [Example 1] Preparation of hollow-fiber-array

A porous plate has holes which have a diameter of 0.32 mm, and a center distance of which holes is 0.42 mm. Specifically, 10 lines were provided lengthwise and breadthwise, respectively, that is, 100 holes are arrayed in total. A sheet of the porous plate with a thickness of 0.1 mm was superposed on another sheet of the porous plate. Through each hole of the two sheets of the porous plates, 100 hollow fibers made of polyethylene (with an external diameter of approximately 300 µm, internal diameter of approximately 160 µm, and length of approximately 50 cm) (MITSUBISHI RAYON CO., LTD.) were passed. The distance between the 2 sheets of the porous plates was determined to be 30 cm. The hollow fibers were kept strained and fixed at two positions: a position 10 cm away from and a position 40 cm away from one end of the hollow fibers.

Next, resin materials were poured between 2 sheets of the porous plates. As the resin, a polyurethane resin adhesive (NIPPON POLYURETHAN INDUSTRY CO., LTD., NIPPOLLAN 4276 and CORONATE 4403) was used after adding carbon black, which was 2.5 % by mass of the total weight of the adhesive, to the adhesive. The resins were hardened by allowing the resultant to stand at room temperature for 1 week. Next, the porous plates were removed, so that a hollow-fiber-array was obtained.

### [Example 2] Introduction and immobilization of polymer gel to hollow-fiber-array

A mixed solution consisting of the following compositions was prepared.

| | |
|---|---|
| Acrylamide | 3.7 parts by mass |
| Methylene bisacrylamide | 0.3 parts by mass |
| 2,2'-azobis (2-amidinopropane) dihydrochloride | 0.1 parts by mass |

The mixed solution and the hollow-fiber-array obtained in Example 1 were placed in a desiccator. The non-arrayed end parts (free end parts) of the hollow-fiber-arrays were immersed in the mixed solution, and then the desiccator was reduced to pressure to introduce the mixed solution was introduced into the hollow portions of the hollow fibers. Subsequently, the hollow-fiber-array was transferred to a sealed glass container having an interior saturated with water vapor, followed by a polymerization reaction at 80°C for 4 hours. Thus, a hollow-fiber-array wherein gelatinized products were immobilized in the hollow portions was obtained. As a result, a hollow-fiber-array holding acrylamide gel inside was obtained.

### [Example 3] Preparation of hollow-fiber-array thin section containing gel

The hollow-fiber-array containing acrylamide gel obtained in Example 2 was sliced in a direction perpendicular to the fiber axis to have a thickness of approximately 2 mm using a micrtome, thereby obtaining a hollow-fiber-array thin section on which 10-by-10 (length-to-width), that is, 100 bundles in total, of the hollow fibers containing gel were regularly arrayed to form a square (Fig. 1). Fig. 1 shows the hollow-fiber-array thin section containing gel as prepared through Examples 1, 2, and 3. The hollow portions 11 of the hollow fibers 12 were filled with the gel prepared in Example 2.

### [Example 4] Example of screening for crystallization conditions utilizing hollow-fiber-array thin section containing gel, and protein crystallization in gel

The hollow-fiber-array thin section containing gel (the microarray) obtained in Example 3 was freeze-dried. One bundle of the hollow fibers of the microarray was selected, to which 1 µL of a 2 mol/L sodium chloride aqueous solution was added dropwise as a protein crystallization agent (spot A). Subsequently, to other bundles of the hollow fibers on the same microarray, crystallization agents shown below were each added dropwise (spots B, C, D, E, F, G, H, I, and J) in a manner similar to the above procedure. The resultants were allowed to stand for several minutes. The protein crystallization agents were held by infiltrating these solutions into the gel.

### <Crystallization agents>

Spot A: 2.0 mol/L sodium chloride aqueous solution
Spot B: 0.5 mol/L sodium chloride aqueous solution
Spot C: 10 % by volume polyethylene glycol (molecular weight of 400) aqueous solution
Spot D: 20 % by volume polyethylene glycol (molecular weight of 400) aqueous solution
Spot E: 10 % by volume polyethylene glycol (molecular weight of 6000) aqueous solution
Spot F: 20 % by volume polyethylene glycol (molecular weight of 6000) aqueous solution
Spot G: 20 % by volume 2-methyl 2,4-pentanediol aqueous solution
Spot H: 40 % by volume 2-methyl 2,4-pentanediol aqueous solution
Spot I: 0.5 mol/L ammonium sulfate aqueous solution
Spot J: 1.5 mol/L ammonium sulfate aqueous solution

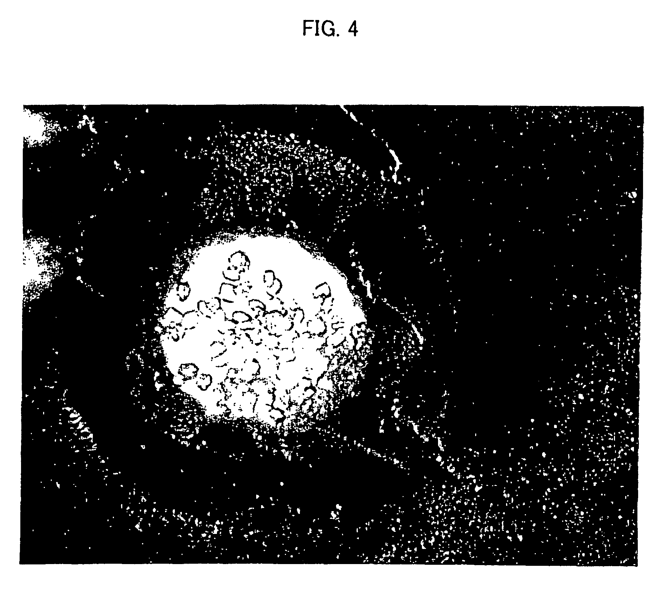

10 µL of an aqueous solution (80 mg/ml) of lysozyme (manufactured by Sigma-Aldrich Corporation) which is an enzyme that degrades polysaccharides, was spotted on the microarray using a 20 µL auto pipette, and then dispersed uniformly on the microarray. The resultant was allowed to stand at 20°C for 3 hours. As a result of observation under an optical microscope, no crystals of the lysozyme were observed at the spot B that had been infiltrated with the polyethylene glycol aqueous solution, and columnar crystals that are optimum for X-ray structural analysis were observed at the spot A that had been infiltrated with the sodium chloride aqueous solution (2 mol/L). (Fig. 4).

Next, based on the above results, approximately 0.3 mm × 0.3 mm × 0.5 mm lysozyme crystals were obtained by the hanging drop vapor diffusion method from the lysozyme-containing sample solution using 2 mol/L sodium chloride as a precipitating agent.

According to the above results, screening was performed using sodium chloride, polyethylene glycol, 2-methyl-2,4-pentanediol, and ammonium sulfate, revealing that sodium chloride at a concentration of 2 mol/L is a condition appropriate for protein crystallization.

Furthermore, as shown in Fig. 4, by crystallizing protein in the gel holding the protein crystallization agents according to the present invention, columnar crystals that are optimum for X-ray structural analysis were obtained. Hence, it was shown that the method of crystallizing protein of the present invention is a convenient and useful method of crystallizing protein and screening therefor.

### INDUSTRIAL APPLICABILITY

According to the present invention, a method of precipitating protein crystals from a protein-containing sample, a novel microarray, and a novel device for screening for conditions for precipitating protein crystals are provided. By the present protein crystallization method, crystals with good quality can be obtained conveniently and highly efficiently. Moreover, by the use of the microarray or the device having crystallization conditions highly integrated therein, protein crystallization conditions can be screened for conveniently and quickly even with an extremely small quantity of a sample.

## Claims

1. A method of crystallizing protein, which comprises the following steps of:
(a) applying a protein-containing sample to polymer gel holding protein crystallization agents; and
(b) bringing the protein into contact with the protein crystallization agents.

2. The method of crystallizing protein of claim 1, wherein the protein-containing sample contains protein, a protein solubilizing agent, and/or an additive.

3. The method of crystallizing protein of claim 1, wherein the protein crystallization agent contains a precipitating agent and/or a pH buffering agent.

4. The method of crystallizing protein of claim 1, wherein the polymer gel holding the protein crystallization agents is transparent.

5. A microarray for protein crystallization, which comprises at least 10 different types and/or different concentrations of protein crystallization agents held thereon per 1 cm² of the microarray surface.

6. The microarray of claim 5, wherein the protein crystallization agent contain a precipitating agent and/or a pH buffering agent.

7. The microarray of claim 5 or 6, wherein the protein crystallization agents are held by means of the polymer gel.

8. The microarray of claim 7, wherein the polymer gel holding the protein crystallization agents is transparent.

9. The microarray of claim 5, which comprises a plurality of hollow fibers arrayed therein holding different types and/or different concentrations of the protein crystallization agents in the hollow portions thereof.

10. The microarray of claim 9, wherein the protein crystallization agents are held by means of the polymer gel that fills the hollow portions.

11. The microarray of claim 9 or 10, wherein the protein crystallization agent contains a precipitating agent and/or a pH buffering agent.

12. A device for protein crystallization, which is provided with the following (a) and (b):
(a) a microarray for protein crystallization having protein-crystallization-agent-holding portions that comprise at least 10 different types and/or different concentrations of protein crystallization agents held thereon per 1 cm² of the microarray surface; and
(b) a plate having wells that can be filled with a protein-containing sample, and correspond to each protein-crystallization-agent-holding portion of the microarray (a) for protein crystallization.

13. The device for protein crystallization of claim 12, wherein in (a), the protein crystallization agents are held by means of polymer gel.

14. The device for protein crystallization of claim 12 or 13, wherein in (a), the protein crystallization agent contains a precipitating agent and/or a pH buffering agent.

15. The device for protein crystallization of any one of claims 12 to 14, wherein in (a), the microarray for protein crystallization comprises a plurality of hollow fibers arrayed thereon holding different types and/or different concentrations of the protein crystallization agents in the hollow portions thereof.

16. The device for protein crystallization of any one of claims 12 to 15, wherein in (b), the capacity of the well is 1 µL or less.

17. The device for protein crystallization of any one of claims 12 to 16, wherein in (b), when superposed on the microarray (a) for protein crystallization, the superposed portion corresponding to each protein-crystallization-agent-holding portion of the array is transparent.

18. The device for protein crystallization of any one of claims 12 to 17, wherein in (b), the plate is prepared from an optically transparent material.

19. The device for protein crystallization of claim 18, wherein the plate is made of a transparent resin or glass.

20. The device for protein crystallization of any one of claims 12 to 19, wherein in (b), the plate has a structure in which a plate material perforated regularly is adhered to a substrate.

21. The device for protein crystallization of claim 20, wherein the substrate is made of an optically transparent material.

22. The device for protein crystallization of claim 20 or 21, wherein the substrate is prepared from a slide glass.

23. The device for protein crystallization of any one of claims 20 to 22, wherein the plate material is made of material selected from the group consisting of metal, resin, and rubber.

24. The device for protein crystallization of any one of claims 12 to 23, wherein the microarray (a) for protein crystallization is fixed on a support.

25. The device for protein crystallization of claim 24, wherein the support is made of an optically transparent material.

26. The device for protein crystallization of claim 24 to 25, wherein the support is prepared from a slide glass.

27. The device for protein crystallization of any one of claims 24 to 26, wherein marking is made on the support so as to determine a position for fixing the microarray (a) for protein crystallization.

28. The device for protein crystallization of any one of claims 24 to 27, wherein a spacer is placed between the support and the plate (b), the spacer having a space into which the microarray (a) for protein crystallization fixed on the support can be accommodated.

29. The device for protein crystallization of claim 28, wherein the spacer is made of material selected from the group consisting of metal, resin, and rubber.

30. A method of screening for protein crystallization conditions, which comprises a step of precipitating protein by bringing the protein crystallization agents into contact with a protein-containing sample on and/or in the microarray, using the microarray of any one of claims 5 to 11, or the device of any one of claims 12 to 29.

31. The method of claim 30, wherein the protein-containing sample contains protein, a protein solubilizing agent, and/or an additive.

32. The method of claim 30 or 31, which is conducted under a plurality of temperature conditions.

33. The method of any one of claims 30 to 32, wherein the volume of the protein-containing sample solution is 1 µL or less per type or concentration of the protein crystallization agent.

34. A method of screening for protein crystallization conditions, which comprises a step of precipitating protein by superposing the wells of (b) filled with the protein-containing sample onto the protein-crystallization-agent-holding portions holding the protein crystallization agents on the microarray (a), so as to bring the protein crystallization agents into contact with the protein in the device for protein crystallization of any one of claims 12 to 29.
